# EUROPÄISCHE PATENTSCHRIFT

(11) **EP 2 845 548 B1**
(45) Veröffentlichungstag und Bekanntmachung des Hinweises auf die Patenterteilung: **16.11.2016**
(21) Anmeldenummer: 13184189.2
(22) Anmeldetag: 12.09.2013
(51) Int. Cl.: A61B 17/29, A61B 18/14, A61B 17/3209, A61B 17/295

(54) **Chirurgisches Instrument mit schwenkbaren Branchen**
Surgical instrument with pivotable jaws
Instrument chirurgical des mors pivotants

(30) Priorität: 10.09.2013 EP 13183633
(43) Veröffentlichungstag der Anmeldung: 11.03.2015
(73) Patentinhaber: ERBE Elektromedizin GmbH, 72072 Tübingen (DE)
(72) Erfinder: Guba, Joachim, 71263 Weil der Stadt (DE); Mitzlaff, Lothar, 8600-531 Lagos (PT); Kühner, Ralf, 70567 Stuttgart (DE); Heim, Martina, 72124 Pliezhausen (DE)
(74) Vertreter: Rüger, Barthelt & Abel

(56) Entgegenhaltungen:
- CN-B- 101 779 979
- DE-B4-102006 062 848
- US-A1- 2002 143 358
- US-A1- 2013 085 516
- US-B1- 6 585 735
- US-B1- 6 818 007

## Beschreibung

Die Erfindung betrifft ein chirurgisches Instrument, insbesondere ein Koagulationsinstrument oder ein Instrument zur Gefäßversiegelung mit einem Werkzeug, das zwei bewegliche Branchen aufweist.

Im Folgenden beschreibt der Begriff "distal" stets den vom Anwender entfernten Teil des Instruments oder Bauteils, der Begriff "proximal" beschreibt stets den zum Anwender hin gerichteten näher liegenden Teil des Instruments oder Bauteils.

Aus US 2013/0085516 A1 geht ein chirurgisches Instrument hervor, dessen Branchen um separate voneinander beabstandete Scharnierachsen schwenkbar gelagert sind. Zusätzlich ist ein längsverschiebbares Messer vorgesehen.

US 6,818,007 B1 offenbart ein chirurgisches Instrument mit schwenkbaren Branchen, die durch ein mit zwei Haken versehenes Zugstück betätigt werden. Die Branche, die eine maulartige Ausnehmung besitzt, reitet dabei auf einem Steg.

DE 10 2006 062 848 B4 offenbart ein chirurgisches Instrument mit zwei voneinander beabstandeten Schwenkachsen bzw. Scharnieren. Die Branchen haben Zapfen die in entsprechende Ausnehmungen greifen. Die Zapfen ergeben jedoch eine andere Lagerung als bei der Erfindung.

Ein Instrument der oben genannten Bauart ist aus der US 2011/0054468 A1 bekannt. Das dort veranschaulichte Instrument weist zwei an dem distalen Ende eines Schafts schwenkbar gelagerte Branchen auf, die ein gemeinsames Schwenklager haben. Das Schwenklager legt eine sich quer zu dem Schaft erstreckende Scharnierachse fest. Zusätzlich ist ein längsverschiebbares Messer vorgesehen, das bei geschlossenen Branchen in distaler Richtung verschoben werden kann, um ein abgeklemmtes und koaguliertes Gefäß zu durchtrennen.

Die US 2003/0199869 A1 offenbart ein ähnliches Instrument mit zwei um eine gemeinsame Scharnierachse schwenkbar gelagerten Branchen. Die Branchen tragen Elektroden zum Koagulieren eines Gefäßes. Wiederum ist ein Messer vorgesehen, das zwischen den geschlossenen Branchen in distaler Richtung verschiebbar ist, um ein koaguliertes und versiegeltes Gewebebündel zu durchtrennen.

In ähnlicher Weise ist ein Instrument nach zumindest einer Ausführungsform der DE 20 2007 009 165 U1 ausgebildet.

Bei allen genannten Konstruktionen ist die Anordnung des Scharnierbereichs mit den Scharnierachsen und des sich durch diesen Scharnierbereich erstreckenden Messers problematisch.

Davon ausgehend ist es Aufgabe der Erfindung, ein Instrument anzugeben, bei dem die beiden Branchen und, falls vorhanden, das Messer mit hoher Präzision geführt ist.

Diese Aufgabe wird mit dem Instrument nach Anspruch 1 gelöst:
Das erfindungsgemäße chirurgische Instrument weist ein Werkzeug mit zwei schwenkbar gelagerten Branchen auf. Jede Branche besteht vorzugsweise aus einem Branchenträger und den darauf fixierten Elektrodeneinheiten. Die Branchenträger dienen dabei zur Übertragung der mechanischen Kräfte und zur Lagerung der Branche. Beide Branchenträger sind an einem Sockelteil an verschiedenen und voneinander beabstandeten Scharnieren aufeinander zu und voneinander weg schwenkbar gehalten. Durch die räumliche Beabstandung der beiden Scharniere und deren Scharnierachsen voneinander, kann zwischen diesen ein Messer angeordnet und gelagert sein.

Die Beabstandung der beiden Scharniere schafft die Möglichkeit einer präziseren Schwenkführung für jede der Branchen durch Vergrößerung der Länge der Scharnierachsen bzw. deren axialer Führungslängen im Vergleich zu einer gemeinsamen Scharnierachse. Die axiale Führungslänge kann mehr als die Hälfte der Breite des Werkzeugs, oder des Durchmessers des Schafts, einnehmen. Die Breite wird dabei in Richtung der Scharnierachse, also quer zum Schaft gemessen. Zusätzlich ermöglicht die Beabstandung der Scharnierachsen, dass bei geöffneten Branchen ein vergrößerter Gewebeaufnahmebereich zur Verfügung steht. Darüber hinaus verändern sich bei Beabstandung der Scharnierachsen die Verhältnisse bezüglich der Kraft zum Schließen der Branchen. Dies ermöglicht größere Schließkräfte, welche zum verbesserten Halten des Gewebes und zur verbesserten Gefäßversiegelung beitragen.

Außerdem kann der zwischen den beiden Scharnieren geschaffene Abstand dazu genutzt werden, den Platz für einen Durchgang für ein Messer zu schaffen. Dieses Messer verläuft somit durch keine der Scharnierachsen. Weiterhin kann in dem Sockelteil ein Schlitz zur Führung für dieses Messer vorgesehen sein, welcher den Durchbruch für das Messer schafft und ein Teil der Führung bzw. Lagerung des Messers übernimmt und diese verbessert.

Zur Ausbildung der genannten Scharniere werden an einem Teil ein Lagerzapfen und an einem anderen Teil eine Lagerbuchse genutzt. Der Lagerzapfen umfasst vorzugsweise ein mit einem Steg verbundenen zylindrischen Lagerabschnitt. Die Lagerbuchse am anderen Teil umfasst einen dazu passenden vorzugweise zylindrischen Abschnitt mit einer Öffnung. Bei einer Ausführungsform ist die Lagerbuchse in dem Sockelteil und der Lagerzapfen an dem Branchenträger der Branche angeordnet. Bei einer nicht erfindungsgemässen Ausführungsform kann die Zuordnung umgekehrt sein.

Der Lagerzapfen ist in Form eines Lagerabschnitts mit Steg ausgebildet, der entlang eines streifenförmigen taillierten Bereichs entlang seiner Mantelfläche mit dem Branchenträger nahtlos einstückig ausgebildet ist. Außerdem geht der Lagerzapfen an einer Stirnseite in den Branchenträger über . Auf diese Weise wird zusammen mit der komplementär geformten Lagerbuchse in dem Sockelteil ein robustes und präzises Scharnier ausgebildet. Das Scharnier ist zudem einfach zu montieren, indem der Lagerzapfen axial in die an einem Ende offene Lagerbuchse eingeschoben wird.

Die Öffnung der Lagerbuchse weist eine Weite auf, die geringer ist als der Durchmesser des zylindrischen Lagerabschnitts des Lagerzapfens. Die Weite der Öffnung ist jedoch etwas größer als der Steg des Lagerzapfens, welcher den Lagerabschnitt mit dem Branchenträger verbindet, so dass eine Schwenkbewegung des Branchenträgers relativ zum Sockelteil möglich ist.

Vorzugsweise ist das Sockelteil aus Kunststoff ausgebildet. Der Branchenträger kann aus Kunststoff, Keramik, Verbundmaterial oder Metall ausgebildet sein. Die Branchenträger können Elektrodeneinheiten tragen, die aus einem Kunststoffkörper mit einem Metallinlay bestehen. Diese Metallinlays können Blechteile sein, welche als Elektroden bzw. Gewebekontaktflächen dienen und durch den Kunststoffkörper von den Branchenträgern elektrisch und thermisch isoliert sind. Damit werden Koagulationswirkungen auf das zwischen den beiden Branchen des Werkzeugs eingeklemmte Gewebe beschränkt.

Eine besonders einfache Montage des Werkzeugs wird ermöglicht, wenn die Branchenträger mit Querarretierungsmitteln versehen sind. Diese arretieren die Branchenträger in Richtung der Scharnierachsen, also quer zum Werkzeug. Solche Querarretierungsmittel können durch an den Branchenträgern ausgebildete, sich in Umfangsrichtung erstreckende Vorsprünge bzw. Rippen und Ausnehmungen bzw. Taschen gebildet sein, die in geschlossenem und normal geöffnetem Zustand des Werkzeugs ineinander greifen. Die Montage der Branchenträger an dem Sockelteil erfolgt dann in einer Montageposition, indem die Branchenträger mit ihren Lagerzapfen in entgegen gesetzten Richtungen in die zueinander parallelen Lagerbuchsen des Sockelteils eingeschoben und dann so aufeinander zu geschwenkt werden, dass jeweils eine Rippe eines Branchenträgers in die zugeordnete Tasche des anderen Branchenträgers greift. Um dies zu bewerkstelligen, ist es vorteilhaft, wenn die Taschen und die Rippen sowohl in Seitenrichtung (axial zur Scharnierachse) wie auch in Umfangsrichtung gegeneinander versetzt sind.

Weitere vorteilhafte Einzelheiten von Ausführungsformen der Erfindung ergeben sich aus der Zeichnung, der Beschreibung oder den Ansprüchen.

Die zweiteilige Form des Anspruchs 1 basiert auf US 2013/0085516 A1. Die beanspruchte Erfindung ist durch den beigefügten Anspruch 1 gegeben. Die bevorzugten Ausführungsbeispiele sind in den abhängigen Ansprüchen gegeben.

Es zeigen:
Figur 1 ein erfindungsgemäßes Instrument, in perspektivischer schematisierter Darstellung.
Figur 2 ein Werkzeug des Instruments nach Figur 1 mit 2 Branchen und dem Sockelteil, in schematisierter Perspektivdarstellung.
Figur 3 ein Sockelteil des Werkzeugs nach Figur 2, in gesonderter perspektivischer Darstellung.
Figur 4 einen Branchenträger einer Branche des Werkzeugs nach Figur 2, in gesonderter ausschnittsweiser Perspektivdarstellung.
Figur 5 das Sockelteil nach Figur 3, in ausschnittsweiser größerer Perspektivdarstellung.
Figur 6 der Fassbereich eines erfindungsgemäßen Instruments 1.

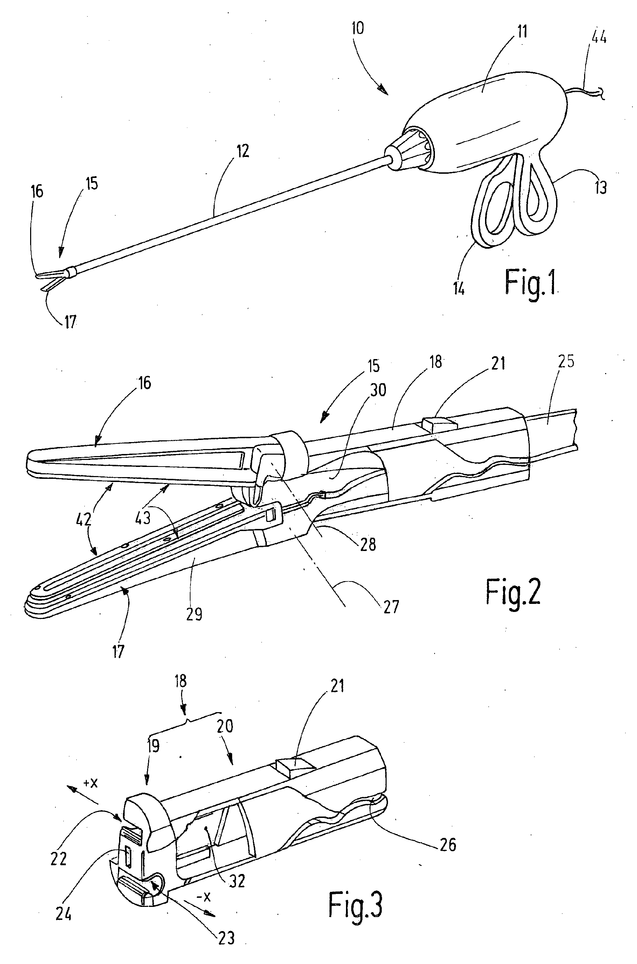

In Figur 1 ist ein chirurgisches Instrument 10 veranschaulicht, das für die offenchirurgische Gefäßversiegelung eingerichtet ist. Das Instrument 10 weist ein Gehäuse 11 auf, von dem sich ein vorzugsweise gerader Schaft 12 weg erstreckt. An dem Gehäuse 11 ist ein Handgriff 13 vorgesehen, in dessen Nachbarschaft ein Bedienhebel 14 schwenkbar gelagert ist. Der Bedienhebel 14 dient zur Betätigung eines Werkzeugs 15, das an dem distalen Ende des Schafts 12 angebracht ist. Das Instrument 10 kann als Einweginstrument ausgebildet und somit für den einmaligen Gebrauch vorgesehen sein. Es kann jedoch auch als sterilisierbares und somit wieder verwendbares Instrument ausgebildet sein.

Die Besonderheit des Instruments 10 liegt in der Ausbildung des Werkzeugs 15, das in Figur 2 gesondert veranschaulicht ist. Das Werkzeug 15 weist eine erste und eine zweite Branche 16, 17 auf, die beide an einem Sockelteil 18 beweglich gelagert sind. Das Sockelteil 18 kann zum Beispiel aus Kunststoff, aus Keramik, aus Verbundmaterial oder auch aus Metall bestehen. In Figur 3 ist das Sockelteil 18 gesondert veranschaulicht. Es weist einen distalen Lagerabschnitt 19 und einen sich davon weg erstreckenden Fortsatz 20 auf. Der Fortsatz 20 wird in das distale Ende des Schafts 12 eingesteckt und beispielsweise mit diesem mittels einer Rastnase 21 verrastet. In dem distalen Lagerabschnitt 19 sind zwei zueinander parallele, zu unterschiedlichen Flanken hin offene hinterschnittene Lagerbuchsen 22, 23 ausgebildet, die sich in Querrichtung +X bzw. -X erstrecken und außerdem zum distalen Ende des Sockelteils 18 hin offen sind. Zwischen den beiden Lagerbuchsen 22, 23 kann ein Schlitz 24 vorgesehen sein, durch den ein Messer 25 zum Durchtrennen koagulierten Gewebes, insbesondere versiegelter Gefäße, geschoben werden kann.

Der innen hohl ausgebildete Fortsatz 20 kann außen an seinen beiden Flanken gewellte Nuten 26 zur Aufnahme elektrischer Leitungen aufweisen, mit denen die Branchen 16, 17 mit Spannung oder Strom versorgt werden. Die gewellten Nuten 26 nehmen die entsprechenden elektrischen Leitungen zugfest auf und wirken somit als Zugentlastung für diese. Die Zugentlastung der elektrischen Leitung kann auch durch anders ausgebildete formschlüssige Mittel wie beispielsweise zueinander versetzte Stift- oder Steganordnungen ausgebildet sein.

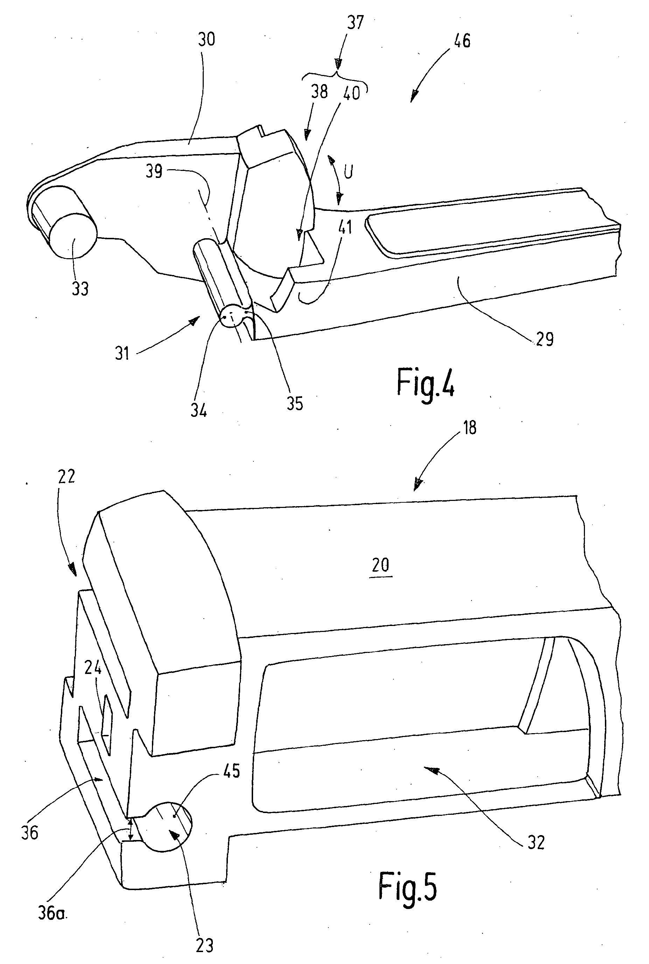

Die Branchen 16, 17 weisen jeweils einen Branchenträger 46 auf, wie er in Figur 4 dargestellt ist. Wie Figur 2 bereits andeutet, sind die beiden Branchen 16, 17 um zwei zueinander parallele voneinander beabstandete Scharnierachsen 27, 28 an dem Sockelteil 18 gelagert. Die entsprechenden Scharniere sind jeweils durch Strukturen des Sockelteils 18 sowie des Branchenträgers 46 der Branche 16, 17 ausgebildet.

Die nachfolgende Erläuterung des Branchenträgers 46 einer ersten Branche 16 gilt entsprechend für die vorzugsweise komplementär ausgebildete zweite Branche 17.

Das die Scharnierachse 27 festlegende Scharnier wird zwischen dem Branchenträger 46 und dem Sockelteil 18 ausgebildet, die in den Figuren 4 und 5 gesondert veranschaulicht sind. Der aus Kunststoff, Keramik, Verbundmaterial oder Metall ausgebildete Branchenträger 46 ist vorzugsweise einstückig nahtlos ausgebildet. In distaler Richtung erstreckt sich ein Werkzeugteil 29, der an seinem hinteren Ende in einen außermittig stehenden Betätigungsteil 30 sowie einen Lagerzapfen 31 übergeht. Der Betätigungsteil 30 passt in eine seitlich in dem Sockelteil 18 vorgesehene Vertiefung mit einem Fenster 32 (Figur 3, Figur 5). Ein an dem Betätigungsteil 30 vorgesehener nach einwärts gerichteter Betätigungszapfen 33 kann dort im Innenraum des Fortsatzes 20 mit Zug-/Schubmitteln zur Schwenkbetätigung des Branchenträgers 46 der ersten Branche 16 in Verbindung stehen. Entsprechendes gilt für die zweite Branche 17.

Der Lagerzapfen 31 weist einen vorzugsweise zylindrischen Lagerabschnitt 34 auf, der über einen Steg 35 mit dem Werkzeugteil 29 verbunden ist. Der Steg 35 erstreckt sich entlang eines Streifens des ansonsten zylindrischen Mantels des Lagerzapfens 31 und geht über die gesamte axiale Länge desselben. Der Lagerzapfen 31 ist ausserdem stirnseitig mit dem Betätigungsteil 30 verwachsen, d.h. einstückig ausgebildet. Der Lagerzapfen 31 ist axial in die Lagerbuchse 23 einschiebbar (Figur 5). Diese Lagerbuchse 23 weist eine Öffnung 36 auf deren Weite 36a geringer ist als der Durchmesser des Lagerabschnitts 34 jedoch größer als die in gleicher Richtung gemessene Dicke des Stegs 35. Dieser schlitzartige Bereich der Lagerbuchse 23 geht in einen zylindrischen Abschnitt 45 über, dessen Durchmesser geringfügig größer ist als der Durchmesser des Lagerabschnitts 34. Dadurch ist eine gewünschte Schwenkbeweglichkeit des Branchenträgers 46 der ersten Branche 16 an dem Sockelteil 18 gegeben. Der maximale Schwenkwinkel ist so groß, dass der zwischen den beiden Branchen 16, 17 gebildete Gewebeaufnahmebereich ausreichend weit geöffnet und geschlossen werden kann. Zu Montagezwecken oder Demontagezwecken können die Branchen 16, 17 auch in einem noch größeren Öffnungswinkel voneinander weg geschwenkt werden.

Entsprechend ist der Branchenträger 46 der zweiten Branche 17 ausgebildet, um mit der Lagerbuchse 22 zusammenzuwirken. Wie aus Figur 5 ersichtlich, ist zwischen den beiden Lagerbuchsen 22, 23 der Schlitz 24 so angeordnet, das er zwischen den beiden Scharnieren verläuft.

Zur seitlichen, d.h. bezüglich dem Lagerzapfen 31 axialen Sicherung der Branchenträger 46 der Branchen 16, 17 aneinander, sind Querarretierungsmittel 37 vorgesehen. Diese können beispielsweise durch an den Branchenträgern 46 der Branchen 16, 17 vorgesehene zueinander komplementäre Strukturen gebildet werden. Zum Beispiel kann an dem Branchenträger 46 in der Nähe des Lagerzapfens 31 seitlich gegen die Mitte des Werkzeugteils 29 versetzt ein Vorsprung beispielsweise in Gestalt einer Rippe 38 vorgesehen sein. Die Rippe 38 kann sich zum Beispiel mit konstantem Radius bezüglich einer zwischen den Scharnierachsen 27, 28 liegende Achse 39 krümmen. Die Rippe 38 erstreckt sich in Umfangsrichtung U (Figur 4) bezüglich dieser Achse 39. Neben der Rippe 38 kann eine Ausnehmung beispielsweise in Gestalt einer Tasche 40 vorgesehen sein, die sich in gleicher Umfangsrichtung erstreckt und zu dem Lagerzapfen 31 hin offen ist. Greift eine komplementäre Rippe des Branchenträgers 46 der zweiten Branche 17 in diese Tasche 40, ist diese andere Rippe zwischen der aus Figur 4 ersichtlichen Rippe 38 und einer Wange 41 gefangen und somit in Querrichtung gesichert. Die Tasche 40 ist zwischen der Rippe 38 und der Wange 41 ausgebildet.

Zur Montage der beiden Branchenträger 46 der Branchen 16, 17 an dem Sockelteil 18, werden die weit voneinander weg gespreizten Branchenträger 46 der Branchen 16, 17 mit ihren entsprechenden Lagerzapfen 31 in die Lagerbuchsen 22, 23 eingeschoben. Sie werden dann etwas aufeinander zu bewegt, so dass die jeweilige Rippe 38 in die ihr zugeordnete, im gegenüberliegenden Branchenträger 46 ausgebildete Tasche 40 greift. Sobald dies geschieht, sind die Branchen 16, 17 aneinander gesichert und können von dem Sockelteil 18 nicht mehr abfallen. Es genügt, wenn an einem Branchenträger 46 der ersten Branche 16 eine Rippe 38 und am Branchenträger 46 der zweiten Branche 17 die Tasche 40 ausgebildet sind. Es ist aber von Vorteil, wenn beide Branchenträger 46 der ersten und der zweiten Branche 16, 17 jeweils mit Rippe 38 und Tasche 40 ausgestattet sind.

An den Branchenträgern 46 der Branchen 16, 17, insbesondere deren Werkzeugteilen 29, sind die aus Figur 2 ersichtlichen Elektrodeneinheiten 42 befestigt, die eine zentrale Messerführungsnut 43 aufweisen können. Bei geschlossenen Branchen 16, 17 fluchtet dieser mit dem Schlitz 24. Ein distal vorgeschobenes Messer 25, das von dem Schlitz 24 präzise geführt wird, kann nun in der Messerführungsnut 43 vom proximalen zum distalen Ende der Elektrodeneinheiten 42 hin vorgeschoben werden.

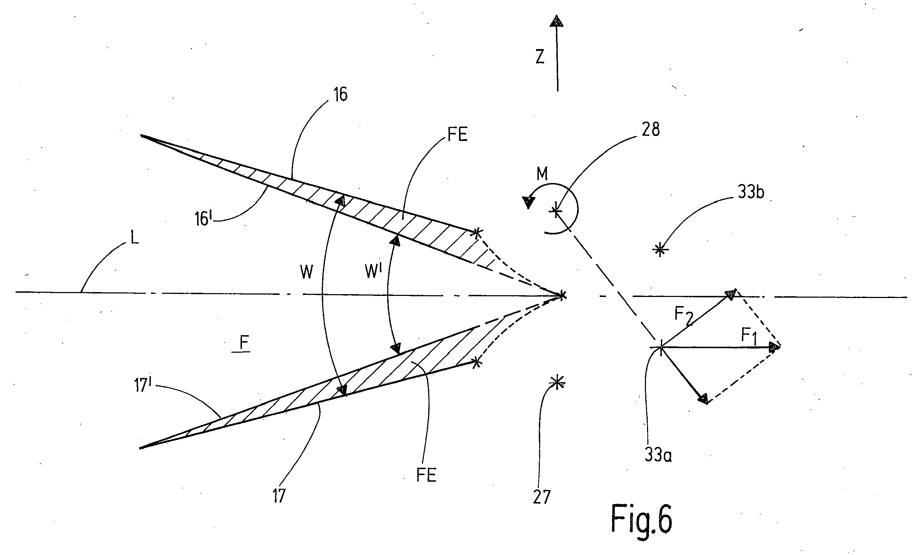

Wie Figur 6 zeigt, vergrößert sich der Fassbereich F des Gewebes zwischen den beiden Branchen 16, 17 aufgrund der zueinander beabstandet angeordneten Scharnierachsen 27, 28 um den Bereich FE. Der Bereich FE ist in Figur 6 schraffiert dargestellt. Die zur Längsachse L des Instruments 10 beabstandet angeordneten Scharnierachsen 27, 28 bewirken, dass der Öffnungswinkel W des Gewebekontaktbereichs der Branchen 16, 17 flacher ausgebildet ist im Vergleich zu einem Gewebekontaktbereich zweier Branchen, deren Scharnierachsen auf der Längsachse L des Instruments angeordnet sind. Der steilere Öffnungswinkel W' ist in Figur 6 zwischen zwei Branchen 16' und 17' eingetragen. Es ist deutlich zu erkennen, dass die Verlagerung der Scharnierachsen 27, 28 weg von der Längsachse L mit dem flacheren Öffnungswinkel W einen zusätzlichen Bereich FE zum Fassen von Gewebe bewirkt.

Das scherenartige Schließen von zwei Branchen beim Fassen von Gewebe bewirkt, dass das Gewebe im proximalen Fassbereich stärker geklemmt wird, als das Gewebe im distalen Fassbereich der Branchen. Dies ist dadurch begründet, dass bei geöffneten Branchen der Abstand zwischen den Branchen im proximalen Fassbereich geringer ist, als im distalen Fassbereich. Aufgrund der Verlagerung der Scharnierachsen 27, 28 weg von der Längsachse L des Instrumentes 10 wird wie oben beschrieben, der Öffnungswinkel W der Branchen 16, 17 flacher. Dies hat einen direkten Einfluss auf die Klemmkraft des Gewebes. Es entsteht ein homogeneres Schließverhalten der Branchen 16, 17, da aufgrund des flacheren Öffnungswinkels W der Abstand zwischen den beiden Branchen 16, 17 im proximalen Fassbereich vergrößert wird. Dieses homogenere Schließverhalten der Branchen 16, 17 reduziert den Einfluss der Position innerhalb der Branchen 16, 17, an der das Gewebe gefasst wird, bezogen auf die Klemmkraft.

Eine von der Längsachse L beabstandete Scharnierachse 28 eines Branchenträgers 46 ermöglicht die Anordnung des Betätigungszapfens 33 in dem zur Verfügung stehenden Bauraum mit einem großen Abstand in Z-Richtung zu der Scharnierachse 27, 28. Figur 6 zeigt die Scharnierachse 28 oberhalb der Längsachse L und den dieser Scharnierachse 28 zugeordneten Betätigungszapfen 33a. Über die Zug-/Schubmittel wird die in Richtung der Längsachse L orientierte Zug-/Schubkraft F1, bevorzugt mittels einer Kulisse, in einer Kraftkomponente F2 auf den Betätigungszapfen 33 zur Wirkung gebracht. Dadurch entsteht ein Drehmoment M um die Scharnierachse 28, welches auf die Branche 16 wirkt. Durch dieses Drehmoment M wird Gewebe, das zwischen den beiden Branchen 16, 17 gefasst ist, kraftvoll und sicher gehalten. Dies erhöht die Qualität und die Reproduzierbarkeit der Gewebeversiegelung. Je größer der Abstand in Z-Richtung zwischen der Scharnierachse 28 und dem Betätigungszapfen 33 ist, desto größer ist der für das Drehmoment M relevante Anteil der Kraftkomponente F2. Weist die Zug- /Schubkraft F nur eine ausschließlich auf den Betätigungszapfen 33 wirkende lineare Komponente auf und liegen der Betätigungszapfen 33 und die Scharnierachse 28 auf einer gemeinsamen Achse, wird kein Drehmoment M um die Scharnierachse 28 erzeugt. Die Wirkung des Abstands in Z-Richtung zwischen der Scharnierachse 28 und dem Betätigungszapfen 33a gilt entsprechend für die Scharnierachse 27 und dem Betätigungszapfen 33b.

Das insoweit beschriebene Instrument 10 arbeitet wie folgt:
Der Anwender kann die Branchen 16, 17 über nicht weiter veranschaulichte Zug-/Schubmittel und ein Getriebe durch Bewegung des Bedienhebels 14 aufeinander zu und voneinander weg bewegen. Er fasst nun zwischen den Branchen 16, 17, beispielsweise ein zu koagulierendes bzw. zu versiegelndes und danach zu durchtrennendes Gewebebündel, welches auch Gefäße umfassen kann, und klemmt dieses durch Schließen der Branchen 16, 17 zwischen den Elektrodeneinheiten 42 fest. Über einen nicht weiter dargestellten Schalter kann er nun die Elektrodeneinheiten 42 der Branchen 16, 17 aktivieren. Diese sind über nicht weiter veranschaulichte Leitungen, die sich durch den Schaft 12 erstrecken und eine Leitung 44, die von dem Gehäuse 11 weg führt, mit einem elektrischen Gerät, beispielsweise einem Generator verbunden. Der Generator kann beispielsweise HF-Strom bzw. HF-Spannung an die Elektrodeneinheiten 42 abgeben. Das zwischen den Elektrodeneinheiten 42 gefasste Gewebe wird dadurch koaguliert, ausgetrocknet und versiegelt. Ist dies geschehen, kann durch Betätigung eines Betätigungselements das Messer 25 in distaler Richtung vorgeschoben werden, wobei es dank des Schlitzes 24 und der Messerführungsnut 43 eine präzise Führung erfährt. Mit seiner stirnseitigen Schneidkante durchtrennt das Messer 25 das koaguliert und versiegelte Gewebe und kann danach zurückgezogen werden.

Das Instrument 10 kann als Einweginstrument ausgebildet sein. Es ist auch möglich, lediglich Teile desselben, beispielsweise das Werkzeug 15 und/oder den Schaft 12, als Einweginstrument auszubilden. Es lässt sich dank seines einfachen Aufbaus bedarfsweise jedoch auch als sterilisierbares und wieder verwendbares Instrument ausbilden.

Ein Instrument 10 mit einem besonders einfach aufgebauten Werkzeug 15 weist Branchenträger 46 einer ersten und zweiten Branche 16, 17 auf, die durch voneinander beabstandete Scharniere an einem gemeinsamen Sockelteil 18 gelagert sind. Die Scharnierachsen 27, 28 der Scharniere sind parallel zueinander orientiert und voneinander beabstandet. Zwischen beiden kann ein Schlitz 24 zur präzisen Führung eines Messers 25 vorgesehen sein. Die Branchenträger 46 der Branchen 16, 17 sind in jeweils eigenen Scharnieren spielarm und somit präzise geführt. Sie sind durch Querarretierungsmittel 37 aneinander gehalten, was eine einfache Montage und eine präzise Führung sicherstellt.

### Bezugszeichenliste:

- 10: Instrument
- 11: Gehäuse
- 12: Schaft
- 13: Handgriff
- 14: Bedienhebel
- 15: Werkzeug
- 16, 16': erste Branche
- 17, 17': zweite Branche
- 18: Sockelteil
- 19: Distaler Lagerabschnitt von 18
- 20: Fortsatz
- 21: Rastnase
- 22: erste Lagerbuchse
- 23: zweite Lagerbuchse
- 24: Schlitz
- 25: Messer
- 26: Nut zur zugfesten Lagerung elektrischer Leitungen
- 27, 28: Scharnierachsen
- 29: Werkzeugteil
- 30: Betätigungsteil
- 31: Lagerzapfen
- 32: Fenster
- 33, 33a, 33b: Betätigungszapfen
- 34: Lagerabschnitt
- 35: Steg
- 36: Öffnung
- 36a: Weite von 36
- 37: Querarretierungsmittel
- 38: Rippe, Vorsprung
- 39: Achse
- 40: Tasche, Ausnehmung
- 41: Wange
- 42: Elektrodeneinheiten
- 43: Messerführungsnut
- 44: Leitung
- 45: Zylindrischer Abschnitt
- 46: Branchenträger
- +X: Richtung quer zum Schaft 12
- -X: Gegenrichtung quer zum Schaft 12
- U: Umfangsrichtung zur Achse 39
- F: Fassbereich
- FE: Zusätzlicher Fassbereich
- W, W': Öffnungswinkel zwischen den beiden Branchen
- M: Drehmoment um Scharnierachse
- F1: Zug-/Schubkraft
- F2: Kraftkomponente für Drehmoment M
- Z: Richtung senkrecht auf Längsachse L
- L: Längsachse des Instruments

## Patentansprüche

1. Chirurgisches Instrument (10), insbesondere zur Gewebekoagulation und Gefäßversiegelung,
mit einem Werkzeug (15), das zwei Branchen (16, 17) aufweist, die an einem Sockelteil (18) um zwei verschiedene Scharnierachsen (27, 28) aufeinander zu und voneinander weg schwenkbar gelagert sind, wobei die Scharnierachsen (27, 28):
- voneinander beabstandet und
- ___parallel zueinander ausgerichtet sind, wobei
- die beiden Scharnierachsen (27, 28) durch zwei voneinander beabstandete Scharniere mit je einem Lagerzapfen festgelegt sind, dadurch gennzeichnet, dass
- der Lagerzapfen (31) in Form eines Lagerabschnitts (34) mit Steg ausgebildet ist, der entlang eines streifenförmigen taillierten Bereichs entlang seiner Mantelfläche mit dem Branchenträger nahtlos einstückig ausgebildet ist und an einer Stirnseite in den Branchenträger übergeht.

2. Instrument nach Anspruch 1, **dadurch gekennzeichnet, dass** das Sockelteil (18) zwischen den Scharnierachsen (27, 28) einen Schlitz (24) für ein Messer (25) aufweist

3. Instrument nach einem der vorigen Ansprüche, **dadurch gekennzeichnet, dass** zu dem Scharnier der Lagerzapfen (31) mit dem Lagerabschnitt (34), der zylindrisch ausgebildet ist, und ein sich radial von diesem weg erstreckender Steg (35) sowie eine Lagerbuchse (22, 23) mit einer Öffnung (36) gehören.

4. Instrument nach Anspruch 3, **dadurch gekennzeichnet, dass** der Lagerzapfen (31) an seinem stirnseitigen Ende mit dem Betätigungsteil (30) der Branche (16, 17) nahtlos verbunden ist.

5. Instrument nach Anspruch 3, **dadurch gekennzeichnet, dass** die Öffnung 36 der Lagerbuchse (23) eine Weite (36a) aufweist, die geringer ist als der Durchmesser des Lagerabschnitts (34) und die größer ist als die Dicke des Stegs (35).

6. Instrument nach einem der Ansprüche 3 bis 5, **dadurch gekennzeichnet, dass** der Lagerzapfen (31) an der Branche (16) und der Lagerbuchse (23) in dem Sockelteil (18) vorgesehen ist.

7. Instrument nach einem der vorigen Ansprüche, **dadurch gekennzeichnet, dass** das Sockelteil (18) aus Kunststoff ausgebildet ist.

8. Instrument nach Anspruch 3, **dadurch gekennzeichnet, dass** die Lagerbuchsen (22, 23) der beiden Scharniere zueinander parallel orientiert und stirnseitig in entgegengesetzten Richtungen (+X, -X) offen sind.

9. Instrument nach einem der vorigen Ansprüche, **dadurch gekennzeichnet, dass** zumindest eine der Branchen (16, 17) einen Branchenträger (46) aufweist.

10. Instrument nach einem der vorigen Ansprüche, **dadurch gekennzeichnet, dass** die beiden Branchen (16, 17) ineinandergreifende Querarretierungsmittel (37) aufweisen.

11. Instrument nach Anspruch 10, **dadurch gekennzeichnet, dass** zu den Querarretierungsmitteln (37) wenigstens ein Vorsprung (38) an einer ersten Branche (16) gehört, der in eine Ausnehmung (40) der zweiten Branche (17) ragend ausgebildet ist.

12. Instrument nach Anspruch 11, **dadurch gekennzeichnet, dass** der Vorsprung (38) eine an der Branche (16) bezüglich einer Achse (39) in Umfangsrichtung (U) sich erstreckende Rippe (38) ist.

13. Instrument nach Anspruch 12, **dadurch gekennzeichnet, dass** die Ausnehmung eine an der Branche (16) in gleicher Umfangsrichtung (U) angeordnete Tasche (40) ist.

14. Instrument nach Anspruch 13, **dadurch gekennzeichnet, dass** die Tasche (40) und die Rippe (38) seitlich gegeneinander versetzt sind.

15. Instrument nach Anspruch 13, **dadurch gekennzeichnet, dass** die Tasche (40) und die Rippe (38) in der Umfangsrichtung (U) gegeneinander versetzt sind.

## Claims

1. Surgical instrument (10), in particular for tissue coagulation and vascular sealing, with a tool (15) comprising two branches (16, 17) that are mounted on a base part (18) so they can pivot towards each other and away from each other about two different hinge axes (27, 28), wherein the hinge axes (27, 28):
- are spaced apart from each other and
- oriented parallel to each other, wherein
- the two hinge axes (27, 28) are established by two hinges spaced apart each other and each having a bearing pin,
**characterised in that**
- the bearing pin (31) is configured in the form of a bearing portion (34) with a web which, along a strip-like tapered region along its casing surface, is configured seamlessly integrally with the branch carrier and on its face end transforms into the branch carrier.

2. Instrument according to claim 1, **characterised in that** between the hinge axes (27, 28), the base part (18) has a slot (24) for a cutter (25).

3. Instrument according to one of the preceding claims, **characterised in that** the hinge comprises a bearing pin (31) with the cylindrically configured bearing portion (34), a web (35) extending radially away from this, and a bearing bush (22, 23) with an opening (36).

4. Instrument according to claim 3, **characterised in that** at its face end, the bearing pin (31) is seamlessly connected to the actuating part (30) of the branches (16, 17).

5. Instrument according to claim 3, **characterised in that** the opening (36) of the bearing bush (23) has a width (36a) which is smaller than the diameter of the bearing portion (34) and greater than the thickness of the web (35).

6. Instrument according to any of claims 3 to 5, **characterised in that** the bearing pin (31) is provided on the branch (16) and the bearing bush (23) is provided in the base part (18).

7. Instrument according to any of the preceding claims, **characterised in that** the base part (18) is made of plastic.

8. Instrument according to claim 3, **characterised in that** the bearing bushes (22, 23) of the two hinges are oriented parallel to each other and open at the end faces in opposing directions (+X, -X).

9. Instrument according to any of the preceding claims, **characterised in that** at least one of the branches (16, 17) has a branch carrier (46).

10. Instrument according to any of the preceding claims, **characterised in that** the two branches (16, 17) have transverse locking means (37) which engage in each other.

11. Instrument according to claim 10, **characterised in that** the transverse locking means (37) comprise at least one protrusion (38) on a first branch (16) which is configured extending into a recess (40) of the second branch (17).

12. Instrument according to claim 11, **characterised in that** the protrusion (38) is a rib (38) on the branch (16) extending in the circumferential direction (U) relative to an axis (39).

13. Instrument according to claim 12, **characterised in that** the recess is a pocket (40) arranged on the branch (16) in the same circumferential direction (U).

14. Instrument according to claim 13, **characterised in that** the pocket (40) and the rib (38) are offset laterally to each other.

15. Instrument according to claim 13, **characterised in that** the pocket (40) and the rib (38) are offset to each other in the circumferential direction (U).

## Revendications

1. Instrument chirurgical (10), en particulier pour la coagulation de tissus et le scellement de vaisseaux,
comprenant un outil (15) qui présente deux mors (16, 17) qui sont montés sur un élément socle (18) avec possibilité de pivotement autour de deux axes charnières (27, 28) différents, de manière à pouvoir être rapprochés et éloignés l'un de l'autre, les axes charnières (27, 28) étant :
- espacés l'un de l'autre et
- orientés parallèlement l'un à l'autre, sachant que
- les deux axes charnières (27, 28) sont fixés par deux charnières espacées l'une de l'autre, comportant chacune un tourillon, **caractérisé en ce que**
- le tourillon (31) est réalisé sous la forme d'un tronçon de palier (34) doté d'une aile qui est réalisée d'une seule pièce, sans joint, avec le support de mors, le long d'une zone cintrée en forme de bande, le long de sa surface enveloppe, et se raccorde par une face frontale au support de mors.

2. Instrument selon la revendication 1, **caractérisé en ce que** l'élément socle (18) présente, entre les axes charnières (27, 28), une fente (24) pour un couteau (25).

3. Instrument selon l'une des revendications précédentes, **caractérisé en ce que** le tourillon (31), avec le tronçon de palier (34) qui est réalisé sous une forme cylindrique, et une aile (35), s'étendant radialement à partir de celui-ci, ainsi qu'un coussinet (22, 23), doté d'une ouverture (36), font partie de la charnière.

4. Instrument selon la revendication 3, **caractérisé en ce que** le tourillon (31) est relié sans joint par son extrémité frontale à l'élément d'actionnement (30) du mors (16, 17).

5. Instrument selon la revendication 3, **caractérisé en ce que** l'ouverture (36) du coussinet (23) présente une largeur (36a) qui est plus faible que le diamètre du tronçon de palier (34) et qui est plus grande que l'épaisseur de l'aile (35).

6. Instrument selon l'une des revendications 3 à 5, **caractérisé en ce que** le tourillon (31) est prévu sur le mors (16) et le coussinet (23) dans l'élément socle (18).

7. Instrument selon l'une des revendications précédentes, **caractérisé en ce que** l'élément socle (18) est réalisé en matière plastique.

8. Instrument selon la revendication 3, **caractérisé en ce que** les coussinets (22, 23) des deux charnières sont orientés parallèlement l'un à l'autre et sont ouverts côté frontal dans des sens opposés (+X, - X).

9. Instrument selon l'une des revendications précédentes, **caractérisé en ce qu'**au moins un des mors (16, 17) présente un support de mors (46).

10. Instrument selon l'une des revendications précédentes, **caractérisé en ce que** les deux mors (16, 17) présentent des moyens de verrouillage transversal (37) s'engageant l'un dans l'autre.

11. Instrument selon la revendication 10, **caractérisé en ce que** les moyens de verrouillage transversal (37) comportent au moins une saillie (38) sur un premier mors (16), qui est réalisée de manière à avancer dans un évidement (40) du deuxième mors (17).

12. Instrument selon la revendication 11, **caractérisé en ce que** la saillie (38) est une nervure (38) s'étendant sur le mors (16), dans la direction périphérique (U) par rapport à un axe (39).

13. Instrument selon la revendication 12, **caractérisé en ce que** l'évidement est un logement (40) disposé sur le mors (16), dans la même direction périphérique (U).

14. Instrument selon la revendication 13, **caractérisé en ce que** le logement (40) et la nervure (38) sont décalés latéralement l'un par rapport à l'autre.

15. Instrument selon la revendication 13, **caractérisé en ce que** le logement (40) et la nervure (38) sont décalés l'un par rapport à l'autre dans la direction périphérique (U).
